# EUROPEAN PATENT APPLICATION

(11) **EP 4 180 091 A1**
(43) Date of publication of application: **17.05.2023**
(21) Application number: 20944230.0
(22) Date of filing: 09.07.2020
(51) Int. Cl.: A61P 35/00, A61K 38/05, A61K 31/517

(54) **COMBINATION DRUG FOR TREATING TERMINAL NON-SMALL CELL LUNG CANCER PATIENT WITH BRAIN METASTASIS**

(71) Applicant: Delta-Fly Pharma, Inc., Tokushima-shi Tokushima 771-0116 (JP)
(72) Inventor: ESHIMA Kiyoshi, Tokushima-shi, Tokushima 771-0116 (JP); SUZUKI Tsuneo, Tokyo 168-0081 (JP)
(74) Representative: Mewburn Ellis LLP
(86) International application number: PCT/JP2020/026826
(87) International publication number: WO 2022/009376

(57) **Abstract**

This invention provides a combined pharmaceutical preparation for treating a terminal non-small cell lung cancer patient or a terminal non-small cell lung cancer patient with brain metastasis (e.g., a kit preparation or a combined preparation), wherein the preparation comprises Ubenimex and Afatinib at doses significantly lower than typical doses and exerts therapeutic effects equivalent to or higher than the effects achieved by Afatinib at a typical dose, while rarely causing adverse effects; use of a low dose of Ubenimex and a low dose of Afatinib in the manufacture of the combined pharmaceutical preparation used for treating such patient; and a method for treatment of the cancer comprising administering the combined pharmaceutical preparation to the patient.

## Description

### Technical Field

The present invention relates to a combined pharmaceutical preparation for treating a terminal non-small cell lung cancer patients or terminal non-small cell lung cancer patients with brain metastasis, which comprises, as active ingredients, low doses of Ubenimex and Afatinib.

### Background Art

The brain metastasis rate for patients having terminal (stage III to stage IV) non-small cell lung cancer is very high and is about 30% (Non-Patent Document 1). When the cancer that has metastasized to the brain cannot be removed by surgery, treatment using a gamma knife (a radiation device for stereotactic radiotherapy) or a molecular-targeted drug for cancer is employed, although satisfactory treatment methods have not yet been established.

Afatinib is a molecular-targeted drug for cancer that is effective on non-small cell lung cancer with a mutation in the EGFR (epidermal growth factor receptor) gene. At a typical dose (40 mg/day) thereof, however, adverse effects, such as rashes throughout the body and diarrhea, are likely to be caused, and the effects of suppressing brain metastasis are not sufficient (Non-Patent Document 2).

Ubenimex is a protease inhibitor and has effects of enhancing the immune competence of a cancer patient. At a dose of 30 mg/day, Ubenimex is approved in Japan as "prolonging the survival of a patient by concurrent use with an agent for maintenance/intensive chemotherapy after induction of complete remission in adult acute non-lymphatic leukemia" (Non-Patent Document 3). However, application thereof has not yet been expanded to postoperative adjuvant chemotherapy for squamous cell lung cancer (Non-Patent Document 4).

Also, use of Ubenimex while changing the dose thereof from 30 mg to 60 mg/day to 10 mg/day in combination with a low-dose anticancer drug, a molecular-targeted drug for cancer, and the like is demonstrated to be effective for treatment of colon cancer, breast cancer, lung cancer, bile duct cancer, renal cancer, and the like (Patent Document 1).

While effects of Osimertinib, which is a molecular-targeted drug for cancer (an EGFR inhibitor), on a non-small cell lung cancer patient with brain metastasis have been reported, such effects are not sufficient (Non-Patent Document 5). In addition, treatment with the use of a gamma knife has been reported to be effective for treatment of cancer metastasized to the brain of non-small cell lung cancer patients with brain metastasis (Non-Patent Document 6), although effects of such treatment are not sufficient.

### Prior Art Documents

Patent Documents

Patent Document 1: JP Patent No. 6,023,902

### Non-Patent Documents

Non-Patent Document 1: S. Owen et al., Front. Oncol., Vol. 4, p. 248, 2014
Non-Patent Document 2: The package insert of Giotrif (Revised edition, March, 2020), Japan
Non-Patent Document 3: The package insert of Bestatin (Revised edition 16, August, 2014), Japan
Non-Patent Document 4: Ichinose et al., J. Natl. Cancer Inst., Vol. 95, p. 605, 2003
Non-Patent Document 5: J.-C.Soria et al., NEJM, November 18, 2017
Non-Patent Document 6: D-S.Kong et al., J. Korean Med. Sci., Vol. 21, p. 527, 2006

### Summary of the Invention

### Objects to Be Achieved by the Invention

Patent Document 1 discloses the use of a low dose of Ubenimex or concurrent use of a low dose of Ubenimex with an antitumor agent or a molecular-targeted drug for cancer, for treatment of elderly cancer patients or terminal cancer patients. However, Patent Document 1 does not describe that use of a low dose of Ubenimex in combination with a low dose of Afatinib is effective for treatment of terminal non-small cell lung cancer patients or terminal non-small cell lung cancer patients with brain metastasis.

Accordingly, it is an object of the present invention to provide a novel therapeutic means that can achieve a high disease control rate (DCR) and a high overall response rate (ORR) for terminal non-small cell lung cancer patients or terminal non-small cell lung cancer patients with brain metastasis.

### Means for Achieving the Objects

The present inventors have conducted intensive studies in order to achieve the objects described above. As a result, they discovered that administration of Ubenimex, which has been clinically approved as prolonging the survival of a patient by concurrent use with an agent for maintenance/intensive chemotherapy after induction of complete remission in adult acute non-lymphatic leukemia, in combination with Afatinib, which is a molecular-targeted drug, to a patient at doses significantly lower than the typical doses would exert therapeutic effects on terminal non-small cell lung cancer patients or terminal non-small cell lung cancer patients with brain metastasis, equivalent to or higher than the effects of Afatinib at the typical dose. They also discovered that adverse effects would be suppressed to a significant extent. The present invention is based on such finding.

Specifically, the present invention has the following features.
[1] A combined pharmaceutical preparation for treating a terminal non-small cell lung cancer patient or a terminal non-small cell lung cancer patient with brain metastasis, which comprises, as active ingredients, about 10 mg/day of Ubenimex in a single dose unit and about 20 mg/day of Afatinib in a single dose unit, for adults.
[2] The combined pharmaceutical preparation according to [1], wherein the non-small cell lung cancer has the Del19 or L858R gene mutation in the epidermal growth factor receptor (EGFR).
[3] The combined pharmaceutical preparation according to [1] or [2], which is a kit preparation or a combined preparation containing Ubenimex and Afatinib at the doses indicated above.
[4] The combined pharmaceutical preparation according to [3], which is in an oral administration form, such as a capsule, a tablet, or a granule.
[5] Use of about 10 mg/day of Ubenimex in a single dose unit and about 20 mg/day of Afatinib in a single dose unit for adults in the manufacture of the combined pharmaceutical preparation according to any of [1] to [4] for treating a terminal non-small cell lung cancer patient or a terminal non-small cell lung cancer patient with brain metastasis.
[6] A method for treatment of a terminal non-small cell lung cancer patient or a terminal non-small cell lung cancer patient with brain metastasis, comprising administering the combined pharmaceutical preparation according to any of [1] to [4] once a day to the terminal non-small cell lung cancer patient or the terminal non-small cell lung cancer patient with brain metastasis.

### Brief Description of the Drawings

Fig. 1A shows the backgrounds of terminal non-small cell lung cancer patients with brain metastasis (i.e., gender, age, smoking history, primary or recurring cancer, TNM classification (primary), brain metastasis, and EGFR genetic test results) who had participated in clinical trials.
Fig. 1B shows the backgrounds of terminal non-small cell lung cancer patients without brain metastasis (i.e., gender, age, smoking history, primary or recurring cancer, TNM classification (primary), brain metastasis, and EGFR genetic test results).
Fig. 2 shows the results of comparison of adverse effects appearing at the rate of 5% or higher, among 229 patients having non-small cell lung cancer (all grades, grade 3 or higher) subjected to the phase III clinical trials (P-III) of Afatinib administration (40 mg/day) and 26 patients having non-small cell lung cancer (all grades, grade 3 or higher, see Fig. 1A and Fig. 1B) subjected to the phase II clinical trials (P-II) of Ubenimex (10 mg/day) and Afatinib administration (20 mg/day).

### Embodiments of the Invention

The present invention relates to a combined pharmaceutical preparation for treating a terminal non-small cell lung cancer patient or a terminal non-small cell lung cancer patient with brain metastasis, which comprises, as active ingredients, about 10 mg/day of Ubenimex in a single dose unit and about 20 mg/day of Afatinib in a single dose unit, for adults. Use of the term "about" with regard to the dose of the active ingredients; i.e., Ubenimex and Afatinib, herein indicates that the value may vary within a range of ± 2 mg.

### <Ubenimex>

"Ubenimex" used herein is a generic name of (2S)-2-[(2S,3R)-3-amino-2-hydroxy-4-phenylbutanoylamino]-4-methylpentanoic acid (CAS registry No. 58970-76-6). According to need, it may form a pharmaceutically acceptably salt together with an amino group and/or a carboxyl group.

When Ubenimex forms a salt, the salt thereof with the amino group is, for example, a salt with an organic or inorganic acid, such as acetic acid, citric acid, succinic acid, maleic acid, tartaric acid, or hydrochloric acid. The salt thereof with the carboxyl group is a salt, such as sodium salt, potassium salt, or ammonium salt (i.e., a salt of the carboxyl group with ammonia or an amine).

Ubenimex may be produced in accordance with a conventional technique. For example, Ubenimex produced as a culture/fermentation product of microorganisms (e.g., *Streptomyces olivoreticuli*) can be used (H. Umezawa et al., Journal of Antibiotics, 1976, 29 (1): 97-99). Alternatively, a commercially available product, such as Bestatin^{®} (Nippon Kayaku Co., Ltd.), can be used. Ubenimex is preferably in a free form, which does not form a salt.

Ubenimex is an aminopeptidase inhibitor, which is considered to bind to an aminopeptidase on the surface of immunocompetent cells (e.g., macrophages, T cells, and bone marrow cells) and act in a chain reaction on the immune system network (F. Abe et al., Biotherapy, 1990; 4 (11): 1708-1718). It is presumed that such biological action would induce production of killer T cells to enhance the immune functions of the patient and exert antitumor effects.

Concerning a typical dose of Ubenimex, Non-Patent Document 3 describes that, when Ubenimex is used concurrently with an agent for maintenance/intensive chemotherapy after induction of complete remission in adult acute non-lymphatic leukemia, Ubenimex is administered orally to a patient in an amount of 30 mg once a day. While adverse effects of Ubenimex are known to be relatively few, adverse effects, such as rashes/redness, itch, nausea/vomiting, and appetite loss, are known.

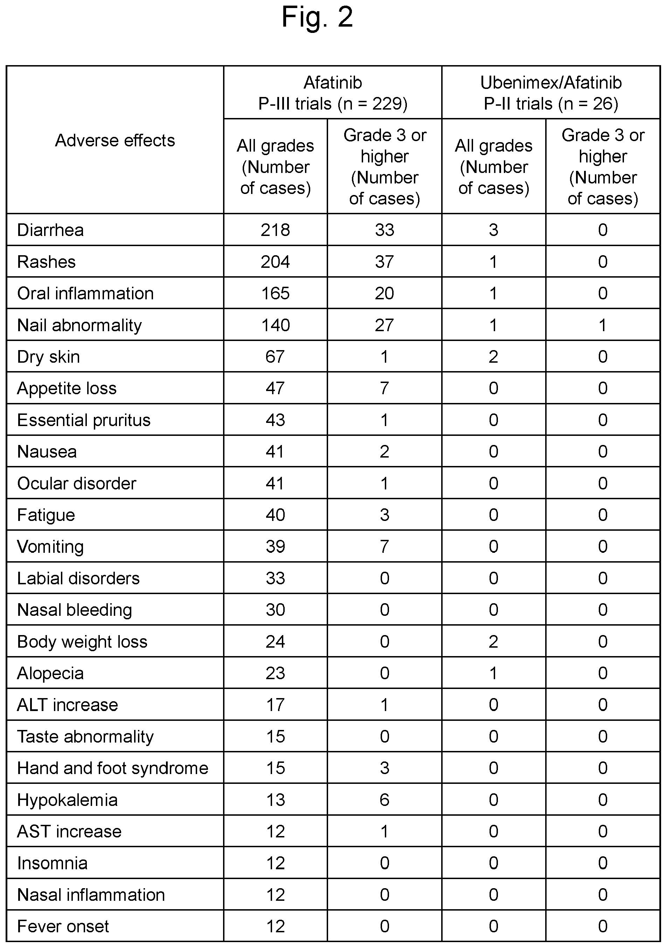

The content of Ubenimex in the combined pharmaceutical preparation according to the present invention corresponds to about 10 mg/day in a single dose unit, for adults. At such dose, Ubenimex is found to cause substantially no adverse effects (Fig. 2).

### <Afatinib>

"Afatinib" used herein is a tyrosine kinase inhibitor, which is known as a second-generation antineoplastic agent used for treatment of non-small cell lung cancer having a specific EGFR gene mutation.

Afatinib is a name of a compound: N-[4-[(3-chloro-4-fluorophenyl)amino]-7-[[(3S)-tetrahydro-3-furanyl]oxy]-6-quinazolinyl]-4-(dimethylamino)-2-butenamide (CAS registry No. 850140-72-6). According to need, Afatinib may form a pharmaceutically acceptable salt with an organic or inorganic acid, such as acetic acid, citric acid, succinic acid, maleic acid, tartaric acid, or hydrochloric acid, or it may not be in the form of a salt. Afatinib is preferably dimaleate.

Methods for synthesizing Afatinib are described in, for example, WO 2007/085638 A1 and WO 2014/183560 A1.

Afatinib is known to exert antitumor effects by competitively inhibiting ATP binding to EGFR/HER1/erbB1, HER2/erbB2, and HER4/erbB4.

Afatinib is commercially available under the trade name "Giotrif" (dimaleate; Boehringer Ingelheim Co., Ltd.) in the form of 20 mg tablets, 30 mg tablets, 40 mg tablets, and 50 mg tablets, and a typical dose of 40 mg is administered once a day (Non-Patent Document 2). Concerning Afatinib, however, adverse effects, such as severe diarrhea, severe skin problems, interstitial pneumonia, and liver failure, are known.

In this regard, the content of Afatinib in the combined pharmaceutical preparation according to the present invention corresponds to about 20 mg/day in a single dose unit for adults. At such dose, Afatinib is verified to cause substantially no adverse effects, such as diarrhea or rashes (Fig. 2).

### <Terminal non-small cell lung cancer patient>

The term "terminal non-small cell lung cancer patient" used herein refers to patient having terminal non-small cell lung cancer, who did not have brain metastasis before subjected to the treatment with the combined pharmaceutical preparation according to the present invention.

A patient with brain metastasis is referred to as a "terminal non-small cell lung cancer patient with brain metastasis" herein.

The term "terminal" used herein refers to a disease stage of cancer, which is stage III to stage IV, and preferably stage IV. Cancer in the terminal stage is diagnosed inoperable by a physician.

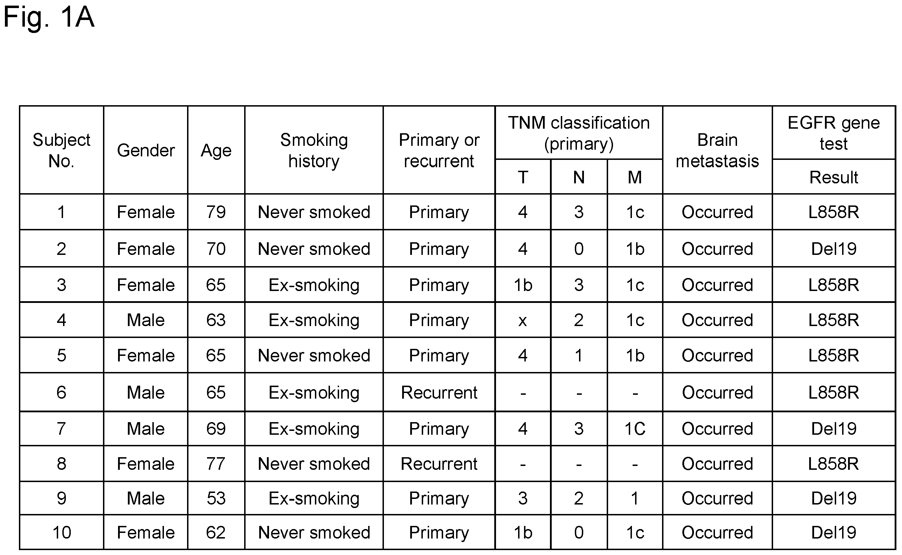

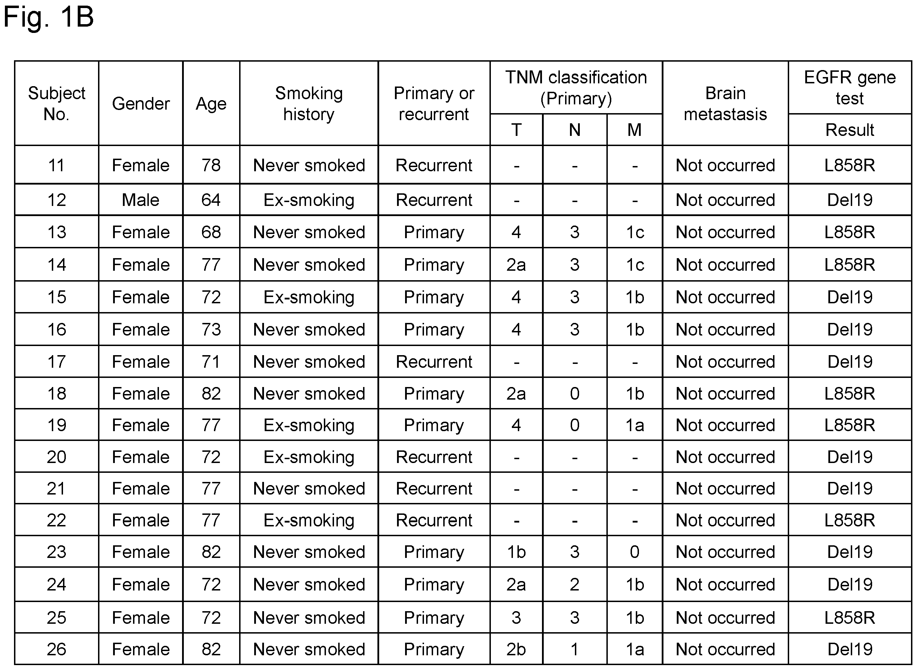

As shown in Fig. 1A and Fig. 1B, the TNM classification is used as an indicator of the degree of progression of malignant tumors (UICC/TNM classification, 8th edition). T indicates the size of the primary tumor, N indicates lymph node metastasis, and M indicates distant metastasis. M0 indicates no distant metastasis (e.g., up to stage IVb), and M1 indicates distant metastasis (e.g., stage IVc).

Even if non-small cell lung cancer patients are in the terminal stage, brain metastasis is not observed in some patients (Fig. 1B), and brain metastasis is observed in some other patients (Fig. 1A). In general, the brain metastasis rate of non-small cell lung cancer is as high as about 30% (Non-Patent Document 1). At present, there is no satisfactory method for treatment of cancer that has metastasized to the brain. In the case of the patients shown in Fig. 1A, for example, they were subjected to a treatment with a gamma knife (a radiation device for stereotactic radiotherapy) before subjected to the treatment according to the present invention, although no therapeutic effects were achieved.

### <Combined pharmaceutical preparation>

The combined pharmaceutical preparation of the present invention comprises, as active ingredients, about 10 mg/day of Ubenimex in a single dose unit and about 20 mg/day of Afatinib in a single dose unit, for adults.

In the treatment of a patient with adult acute non-lymphatic leukemia with the use of Ubenimex, in general, Ubenimex (i.e., Bestatin^{®}) is administered to an adult in an amount of 30 mg (e.g., 10 mg × 3) per day. In the treatment of a non-small cell lung cancer patient with the use of Afatinib, in general, Afatinib (i.e., Giotrif^{®}) is administered to an adult in an amount of 40 mg or 50 mg per day.

As described above, the dose of Ubenimex in the combined pharmaceutical preparation of the present invention is about one-third of the typical dose, and the dose of Afatinib is about one-half of the typical dose. At such low doses, the combined pharmaceutical preparation of the present invention can exert therapeutic effects on terminal non-small cell lung cancer and cancer metastasized to the brain of patients with terminal non-small cell lung cancer with or without brain metastasis (26 patients, Fig. 1A and Fig. 1B); i.e., the disease control rate (DCR) of 100% (26 patients/26 patients), and such effects are equivalent to or higher than the effects of conventional treatment techniques. In addition, the combined pharmaceutical preparation can yield the overall response rate (ORR) of 65% or higher (17 patients/26 patients), and such effects are equivalent to or higher than the effects of conventional treatment techniques. Thus, the combined pharmaceutical preparation of the present invention can provide excellent medical effects. In the examples described below, brain metastasis was not observed during the period of treatment of terminal non-small cell lung cancer patients without brain metastasis. It could be presumed that the brain metastasis was suppressed. During the period of treatment of terminal non-small cell lung cancer patient with brain metastasis, the brain tumor size was not increased.

As shown in Fig. 2, in addition, adverse effects, such as diarrhea and rashes, are likely to appear in the treatment of terminal (stage III to IV) non-small cell lung cancer patients with the use of Afatinib (40 mg/day). According to the present invention, in contrast, substantially no adverse effects, such as diarrhea and rashes, are observed in the treatment of terminal (stage III to stage IV) non-small cell lung cancer patients including cases with brain metastasis (38.5%), with the use of Afatinib (about 20 mg/day) in combination with Ubenimex (about 10 mg/day). In particular, grade 3 or higher diarrhea or rashes are not observed at all.

Concerning the mutation of the epidermal growth factor receptor (EGFR) gene in non-small cell lung cancer, the results of genetic testing performed on the patients who had participated in clinical trials (Fig. 1A and Fig. 1B) demonstrate that there is the Del19 or L858R mutation in EGFR. This indicates that the combined pharmaceutical preparation of the present invention is effective for treatment of terminal non-small cell lung cancer patients having the gene mutation described above (with or without brain metastasis).

The form of the combined pharmaceutical preparation of the present invention is not limited, as long as it contains Ubenimex and Afatinib at the doses indicated above. For example, such form is a kit preparation or a combined preparation.

The kit preparation is a pharmaceutical product comprising multiple units (e.g., 10 to 30 units) of each of a pharmaceutical composition containing about 10 mg/day of Ubenimex in a single dose unit and a pharmaceutical composition containing about 20 mg/day of Afatinib in a single dose unit, for adults, packaged in a single package.

A combined preparation is a pharmaceutical composition containing about 10 mg/day of Ubenimex in a single dose unit and about 20 mg/day of Afatinib in a single dose unit, for adults (i.e., the weight ratio of Ubenimex:Afatinib: about 1:2).

The pharmaceutical composition described above may comprise, in addition to the active ingredient; i.e., Ubenimex or a pharmaceutically acceptable salt thereof, and/or another active ingredient; i.e., Afatinib or a pharmaceutically acceptable salt thereof, agents that are commonly used in a pharmaceutical production, such as an excipient, a binder, a disintegrator, a lubricant, a diluent, a solubilizer, a suspending agent, an isotonizing agent, a pH modifier, a buffer, a stabilizer, a colorant, a taste-improving agent, or a flavoring agent. The pharmaceutical composition can be prepared in a dosage form suitable for oral or parenteral administration (e.g., intravenous administration, intraarterial administration, topical administration by injection, intraperitoneal or intrathoracic administration, transpulmonary administration, subcutaneous administration, intramuscular administration, sublingual administration, percutaneous absorption, or intrarectal administration), with oral administration being preferable. Examples of dosage forms of the pharmaceutical composition of the present invention include, but are not limited to, a solution, an emulsion, a liposome preparation, an injection, a suspension, an ointment, a cream, a transdermal absorbent, a transmucosal absorbent, a tablet, a pill, a capsule, a powdered medicine, a powder, a granule agent, a fine granule agent, and a syrup, with the capsule, tablet, or granule agent being preferable (the oral dosage form is preferable). Such agents can be formulated, molded, or prepared in accordance with conventional techniques in the art.

The effects of the combined pharmaceutical preparation of the present invention on a patient to which the pharmaceutical preparation had been administered can be evaluated with the use of at least one of the following indicators (i) to (iv), in comparison with a patient to which the pharmaceutical preparation had not been administered or a patient before subjected to the administration of the pharmaceutical preparation:
(i) non-small cell lung cancer and, according to need, cancer metastasized to the brain have shrunk or disappeared or would not be increased;
(ii) the prolonged survival (life-extending effect) is observed;
(iii) active ingredients cause no or substantially no adverse effects; and
(iv) metastasis to the brain is not observed or metastasis is suppressed.

When pharmaceutical compositions included in the package formulation of the present invention are concurrently administered to a patient, a pharmaceutical composition containing Ubenimex and a pharmaceutical composition containing Afatinib can be administered continuously or intermittently, to the extent that active ingredients can exert their effects simultaneously. In such a case, the form of administration, the route of administration, and the means of administration of the pharmaceutical compositions may be the same with or different from each other.

When the combined pharmaceutical preparation of the present invention is administered to a patient, the frequency of administration of the combined pharmaceutical preparation of the present invention to a patient can be, for example, 1 to 3 times a day, once a day, or every 2 to 10 days, depending on factors, such as the patient's age, body weight, disease severity, and adverse effects, to the extent that the daily doses of the active ingredients are within the doses indicated above. In such a case, the administration conditions, such as the frequency of administration, the intervals of administration, and the number of days of administration, can be modified by a physician.

Examples of adverse effects include diarrhea, rashes, mouth inflammation, nail abnormality, dry skin, and appetite loss, as exemplified in Fig. 2.

### <Manufacture of the combined pharmaceutical preparation>

The present invention also relates to use of about 10 mg/day of Ubenimex in a single dose unit and about 20 mg/day of Afatinib in a single dose unit for adults, in the manufacture of the combined pharmaceutical preparation for treating terminal non-small cell lung cancer patients or terminal non-small cell lung cancer patients with brain metastasis.

The pharmaceutical preparation can be prepared in a predetermined dosage form by compounding predetermined amounts of each of the active ingredients; i.e., Ubenimex and Afatinib, with above-mentioned pharmaceutically acceptable excipients and additives. Concerning formulation of the pharmaceutical preparation, a reference can be made to, for example, the disclosure of Remington: The Science and Practice of Pharmacy, 23rd Edition, 2012 (Academic Press, U.S.A.).

### <Method for treatment>

The present invention also relates to a method for treatment of a terminal non-small cell lung cancer patient or a terminal non-small cell lung cancer patient with brain metastasis, comprising administering the combined pharmaceutical preparation once a day to the terminal non-small cell lung cancer patient (without brain metastasis) or the terminal non-small cell lung cancer patient with brain metastasis.

In such method, the combined pharmaceutical preparation of the present invention, usage thereof, and conditions for administration thereof, such as the dose, are as described above.

### Examples

Hereafter, the present invention is described in greater detail with reference to the following examples, although the scope of the present invention is not limited to these examples.

### Example 1: Treatment of non-small cell lung cancer patients by concurrent administration of low dose of Ubenimex and low dose of Afatinib

To patients with non-small cell lung cancer having a mutation in the epidermal growth factor receptor (EGFR) gene, 10 mg/day of Ubenimex in a single dose unit (free form) and 20 mg/day of Afatinib (Giotrif^{®}, dimaleate) in a single dose unit were administered in concurrently once a day until no antitumor effects would be exerted. The therapeutic effects and reduction of adverse effects were then inspected.

### <Backgrounds of subjects>

The backgrounds of subjects (26 subjects) participated in the clinical trials (phase-II trials) are as described below (Fig. 1A and Fig. 1B).
(1) Non-small cell lung cancer patients with brain metastasis, on which the treatment with a gamma knife (a radiation device for stereotactic radiotherapy) was not effective: 10 subjects
(2) Non-small cell lung cancer patients without brain metastasis: 16 subjects
(3) Gender: female subjects: 21; male subjects: 5
(4) Age: 53- to 82-year-old (average: 71.7-year-old)
(5) Smoking history: currently smoking: 0 subjects; ex-smoking: 10 subjects; never smoked: 16 subjects
(6) Time of onset: primary: 18 subjects; recurrent: 8 subjects
(7) Brain metastasis: occurred: 10 subjects (Fig. 1A); not occurred: 16 subjects (Fig. 1) (the brain metastasis rate: 38.5%)
(8) Gene mutation type: Del19 mutation: 13 subjects; L858R mutation: 13 subjects

### <Evaluation method>

### 1. Disease control rate (DCR)

DCR is an index concerning the control of clinical conditions of a cancer patient, and it encompasses not only the effects of reducing a cancer size but also the invariance of the cancer size.

Specifically, DCR is determined by ratios of CR (complete response), PR (partial response), and SD (stable disease) based on RECIST evaluation (Journal of the National Cancer Institute, 2000, Vol. 92, No. 3, 205-216). CR, PR, SD, and PD are evaluated based on the following criteria.
Complete response (CR): Disappearance of cancer is sustained for 4 weeks
Partial response (PR): At least 30% decrease in cancer size and it is sustained for 4 weeks
Stable disease (SD): A state between PR and PD
Progressive disease (PD): At least 20% increase in cancer size

### 2. Overall response rate (ORR)

The overall response rate (ORR) is a rate of cases in which cytoreductive effects were achieved. Specifically, the effects are evaluated based on the total of the complete response (CR) cases in which complete disappearance of lesions was observed by CT (computed tomography) and the partial response (PR) cases in which the tumor diameter was reduced to 70% in RECIST evaluation. Also, the occurrence of metastasis of terminal non-small cell lung cancer to other organs, the size of cancer metastasized to other organs, the occurrence of brain metastasis, and changes in the size of cancer metastasized to the brain are evaluated.

### 3. Adverse effects

Concerning the adverse effects shown in Fig. 2 (e.g., diarrhea, rashes, mouth inflammation, and nail abnormality), the number of cases in which adverse effects appeared at the rate of 5% or higher is determined.

### <Results>

The efficacy achieved by administration of the combined pharmaceutical preparation of the present invention is shown as the result of comprehensive evaluation for terminal non-small cell lung cancer patients (without brain metastasis) and for terminal non-small cell lung cancer patients with brain metastasis.

The disease control rate (DCR) was 100% (26 cases/26 cases).

The overall response rate (ORR) was 65% or higher (17 cases/26 cases). In the terminal non-small cell lung cancer patients without brain metastasis shown in Fig. 1B, brain metastasis was not detected. In the terminal non-small cell lung cancer patients with brain metastasis shown in Fig. 1A, at least an increase in the tumor size was not observed.

In the treatment of terminal (stage III to stage IV) non-small cell lung cancer patients including the cases with brain metastasis (38.5%), by concurrent treatment using Afatinib (20 mg/day) and Ubenimex (10 mg/day), adverse effects, such as diarrhea or rashes, were rarely observed.

### Industrial Applicability

The treatment of terminal non-small cell lung cancer patients and terminal non-small cell lung cancer patients with brain metastasis by administration of a low dose of the combined pharmaceutical preparation according to the present invention can achieve excellent efficacy and remarkably reduce adverse effects. Thus, the present invention is industrially applicable.

All publications, patents and patent applications cited herein are incorporated herein by reference in their entirety.

## Claims

1. A combined pharmaceutical preparation for treating a terminal non-small cell lung cancer patient or a terminal non-small cell lung cancer patient with brain metastasis, which comprises, as active ingredients, about 10 mg/day of Ubenimex in a single dose unit and about 20 mg/day of Afatinib in a single dose unit, for adults.

2. The combined pharmaceutical preparation according to Claim 1, wherein the non-small cell lung cancer has the Del19 or L858R gene mutation in the epidermal growth factor receptor (EGFR).

3. The combined pharmaceutical preparation according to Claim 1 or 2, which is a kit preparation or a combined preparation containing Ubenimex and Afatinib at the doses indicated above.

4. The combined pharmaceutical preparation according to Claim 3, which is in an oral administration form, such as a capsule, a tablet, or a granule.

5. Use of about 10 mg/day of Ubenimex in a single dose unit and about 20 mg/day of Afatinib in a single dose unit, for adults, in the manufacture of the combined pharmaceutical preparation according to any one of Claims 1 to 4 for treating a terminal non-small cell lung cancer patient or a terminal non-small cell lung cancer patient with brain metastasis.

6. A method for treatment of terminal a non-small cell lung cancer patient or a terminal non-small cell lung cancer patient with brain metastasis, comprising administering the combined pharmaceutical preparation according to any one of Claims 1 to 4 once a day to the terminal non-small cell lung cancer patient or the terminal non-small cell lung cancer patient with brain metastasis.
